# EUROPÄISCHE PATENTSCHRIFT

(11) **EP 1 572 685 B1**
(45) Veröffentlichungstag und Bekanntmachung des Hinweises auf die Patenterteilung: **03.01.2007**
(21) Anmeldenummer: 03782241.8
(22) Anmeldetag: 27.11.2003
(51) Int. Cl.: C07D 405/12, A61K 31/404, A61P 25/00

(54) **SUBSTITUIERTE BENZDIOXEPINE**
SUBSTITUTED BENZODIOXEPINS
BENZODIOXEPINES SUBSTITUEES

(30) Priorität: 20.12.2002 EP 02028596
(43) Veröffentlichungstag der Anmeldung: 14.09.2005
(73) Patentinhaber: Merck Patent GmbH, 64293 Darmstadt (DE)
(72) Erfinder: HEINRICH, Timo, 64823 Gross-Umstadt (DE); BÖTTCHER, Henning, 64287 Darmstadt (DE); SCHIEMANN, Kai, 64342 Seeheim-Jugenheim (DE); HÖLZEMANN, Günter, 64342 Seeheim-Jugenheim (DE); VAN AMSTERDAM, Christoph, 64295 Darmstadt (DE); BARTOSZYK, Gerd, 64331 Weiterstadt (DE); GREINER, Hartmut, 64331 Weiterstadt (DE); SEYFRIED, Christoph, 64342 Seeheim-Jugenheim (DE); BRUNET, Michel, F-69780 Toussieu (FR); ZEILLER, Jean, F-69008 Lyon (FR); BERTHELON, Jean, F-69003 Lyon (FR)
(86) Internationale Anmeldenummer: PCT/EP2003/013373
(87) Internationale Veröffentlichungsnummer: WO 2004/058746

(56) Entgegenhaltungen:
- EP-A- 0 769 496
- WO-A-03/087086
- GB-A- 2 007 656

## Beschreibung

Die Erfindung betrifft substituierte Benzdioxepine der Formel I 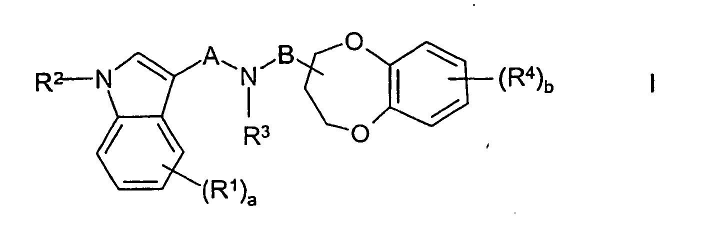 worin
- R¹: unabhängig voneinander ausgewählt ist unter Alkyl, (CH₂)ₘOD, (CH₂)ₘCN, (CH₂)ₘCOR⁵ oder (CH₂)ₘCH₂R⁵ wobei m=0 oder 1 ist
- R², R³: unabhängig voneinander ausgewählt ist unter H, Alkyl mit 1 bis 5 C-Atomen,
- R⁴: unabhängig voneinander ausgewählt ist unter Alkyl mit 1 bis 5 C-Atomen, Heteroalkyl mit 1 bis 5 C-Atomen, Alkoxy mit 1 bis 5 C-Atomen, Alkoxyalkyl mit 2 bis 5 C-Atomen, Hal, CN, COR⁵ oder OH,
- R⁵: für OD, NH₂, NHD oder ND₂,
- A: für CₙH₂ₙ mit n= 2, 3, oder 4,
- B: für CₚH₂ₚ mit p=0, 1, 2, 3 oder 4 steht,
- D: unabhängig voneinander ausgewählt ist unter H, Alkyl mit 1 bis 5 C-Atomen, Alkoxyalkyl mit 2 bis 5 C-Atomen, Aryl oder ArAlkyl,
- a, b: für 0, 1 oder 2 und
- Hal: für F, CI, Br oder I steht,
sowie deren physiologisch verträgliche Salze und Solvate.

Substituierte Benzdioxepine und deren Verwendung als pharmazeutische Wirkstoffe zur Behandlung von Hypertension sind zum Beispiel in DE 2847623, BE 613210, BE 613212 und BE 613215 beschrieben.

Der Erfindung lag die Aufgabe zugrunde, neue Verbindungen mit wertvollen Eigenschaften aufzufinden, insbesondere solche, die sich zur Herstellung von Arzneimitteln eignen.

Es wurde nun gefunden, dass die Verbindungen der Formel I und ihre physiologisch verträglichen Salze und Solvate besonders wertvolle pharmakologische Eigenschaften besitzen. Vor allem zeigen sie besondere Wirkungen auf das Zentralnervensystem, vor allem 5HT-Wiederaufnahme hemmende und 5HT1A-agonistische Wirkungen sowie zum Teil eine sehr hohe Affinität zum 5HT4 Rezeptor-Subtyp. Die erfindungsgemäßen Verbindungen zeigen ferner serotonin-agonistische oder antagonistische Eigenschaften.

Verbindungen der Formel I weisen besonders wertvolle pharmakologische Eigenschaften auf. Die Verbindungen sind besonders für die Herstellung von Arzneimitteln für Anxiolytika, Antidepressiva, Antipsychotika, Neuroleptika und Hypertonika geeignet. Die Verbindungen der Formel I eignen sich zur Behandlung und/oder Prophylaxe verschiedener Erkrankungen des zentralen Nervensystems wie z.B. Schlaganfall, cerebrale Ischämie sowie zur Minderung von Folgeschäden einer Ischämie, Traumata, Hypoglykämie, Schizophrenie, Depression, Demenz, Dyskinesie, neurodegenerativer Erkrankungen wie Parkinsonscher Krankheit, ALS, Morbus Alzheimer, Lewy bodies Dementia oder Huntington Syndrom, Tourette Syndrom, Angst, Lern- und Erinnerungseinschränkungen sowie Schlafstörungen, entzündungsbedingte Hyperalgesie, Himödemen, Unterversorgungszuständen (Hypoxie).

Weiterhin können die Verbindungen der Formel I zur Behandlung und/oder Prophylaxe von entzündlichen Darmerkrankungen und der damit verbundenen Krankheitssymptome, von funktionellen Magen-DarmErkrankungen, die mit Schmerzen und/oder einer vermehrten oder verminderten Peristaltik einhergehen, insbesondere der Irritable bowel Syndroms (IBS) oder zur Behandlung der nicht mit einem Ulkus verbundenen Dyspepsie, Obstipation, insbesondere opiod-induzierte Obstipation, von Arthritis, Migräne, Psoriasis oder anderen juckenden Hauterkrankungen, Dysmenorrhoe und Fibromylagia eingesetzt werden.

Die Verbindungen der Formel I eignen sich auch zur Behandlung und/oder Prophylaxe von Schmerzzuständen, insbesondere von Schmerzüberempfindlichkeits-reaktionen auftretend bei Rückenleiden, Brandverletzungen, Sonnenbrand und rheumatischen Erkrankungen sowie zur Behandlung von postoperativen Schmerzen sowie des häufig nach Abdominaloperationen auftretenden Ileus. Die Verbindungen der Formel I wirken vorzugsweise daüber hinaus analgetisch, antiinflammatorisch, antiasthmatisch, diuretisch, antikonvulsiv, neuroprotektiv und antitussuiv und sind daher vozugsweise geeignet für die Behandlung einer entzündungsbedingten Hyperalgesie, zur Behandlung von Hirnödemen, bei Unterversorgungszuständen (Hypoxie), Schmerzzuständen, sowie zur Minderung der Folgeschäden einer Ischämie.

Weitere Anwendungen finden die Verbindungen der Formel I in der Behandlung und/oder Prophylaxe von Erkrankungen der Blase, insbesondere der Reizblase, auch irritable bladder, Zytalgie, Zystalgie, Neuralgia oder Blasenneurose. Der Begriff Reizblase steht für einen vor allem bei Frauen vorkommenden chronischen Reizzustand des unteren Harntrakts. Symptome sind Dysurie, imperativer Harndrang, Pollakisurie, suprapubische und diffuse Schmerzen beim Sitzen. Häufig besteht eine ausgeprägte Diskrepanz zwischen subjektiven Beschwerden und den objektiven Befunden. Häufigste Ursachen sind Störungen des psychovegetativen oder endokrinen Systems. Die Reizblase ist zu unterscheiden von anderen Krankheitsbildern, wie Harnweginfektionen und Veränderungen des unteren Harntrakts, Erkrankungen benachbarter Beckenorgane oder ZNS oder Rückenmarkerkrankungen (z.B. Multiple Sklerose).

Zum ex-vivo Nachweis der Serotonin-Wiederaufnahmehemmung kann zum Beispiel die synaptosomale Aufnahmehemmung (Wong et al., Neuropsychopharmacol. 8 (1993), 23-33) und der p-Chloramphetaminantagonismus (Fuller et al., J. Pharmacol. Exp. Ther. 212 (1980), 115-119) herangezogen werden.

Bindungseigenschaften der Verbindungen der Formel I lassen sich zum Beispiel durch den 5-HT1A-(Serotonin)-Bindungstests bestimmen (Matzen et al., J. Med. Chem., 43 (2000), 1149-1157, insbesondere Seite 1156 mit Verweis auf Eur. J. Pharmacol.: 140 (1987), 143-155).

Zur Bestimmung der Bindungseigenschaften der Verbindungen der Formel I an den 5-HT4 Rezeptor kann der Test nach Grossman et al. herangezogen werden (Grossman et al., Br. J. Pharmacol. 109, (1993), 618-24).

Gegenstand der Erfindung sind die Verbindungen der Formel I und und/oder deren Enantiomere, Diastereomere, Racemate sowie deren physiologisch verträglichen Salze und Solvate als Liganden der 5 HT1A Rezeptoren und/oder der 5HT4 Rezeptoren mit gleichzeitig starker Serotonin-Wiederaufnahmehemmung.

Gegenstand der Erfindung sind demgemäss die Verbindungen der Formel I und/oder deren Enantiomere, Diastereomere, Racemate sowie deren physiologisch verträglichen Salze und Solvate als Liganden der 5HT1A Rezeptoren und/oder der 5HT4 Rezeptoren mit gleichzeitig starker Serotonin-Wiederaufnahmehemmung zur Behandlung und/oder Prophylaxe verschiedener Erkrankungen wie z.B. Schlaganfall, cerebrale Ischämie sowie Minderung von Folgeschäden einer Ischämie, Traumata, Hypoglykämie, Schizophrenie, Depression, Demenz, Dyskinesie, neurodegenerative Erkrankungen wie Parkinsonsche Krankheit, ALS, Morbus Alzheimer, Lewy bodies Dementia oder Huntington Syndrom, Tourette Syndrom, Angst, Lern- und Erinnerungseinschränkungen, Schlafstörungen, entzündungsbedingte Hyperalgesie, Himödemen, Unterversorgungszuständen (Hypoxie), entzündliche Darmerkrankungen und der damit verbundenen Krankheitssymptome, funktionelle Magen-Darm-Erkrankungen, die mit Schmerzen und/oder einer vermehrten oder verminderten Peristaltik einhergehen, insbesondere des Irritable bowel Syndroms, zur Behandlung und/oder Prophylaxe der nicht mit einem Ulkus verbundenen Dyspepsie, Obstipation, insbesondere opiod-induzierte Obstipation, Arthritis, Migräne, Psoriasis oder anderen juckenden Hauterkrankungen, Dysmenorrhoe, Fibromylagia, Schmerzzuständen, insbesondere von Schmerzüberempfindlichkeitsreaktionen auftretend bei Rückenleiden, Brandverletzungen, Sonnenbrand und rheumatischen Erkrankungen, postoperativen Schmerzen sowie des häufig nach Abdominaloperationen auftretenden Ileus, Erkrankungen der Blase, insbesondere der Reizblase, auch irritable bladder, Zytalgie, Zystalgie, Neuralgia oder Blasenneurose.

Gegenstand der Erfindung ist demgemäss auch die Verwendung der Verbindungen nach Formel I und/oder deren Enantiomere, Diastereomere, Racemate sowie deren physiologisch verträglichen Salze und Solvate zur Herstellung eines Arzneimittels zur Behandlung und/oder Prophylaxe verschiedener Erkrankungen wie z.B. Schlaganfall, cerebrale Ischämie sowie Minderung von Folgeschäden einer Ischämie, Traumata, Hypoglykämie, Schizophrenie, Depression, Demenz, Dyskinesie, neurodegenerätive Erkrankungen wie Parkinsonsche Krankheit, ALS, Morbus Alzheimer, Lewy bodies Dementia oder Huntington Syndrom, Tourette Syndrom, Angst, Lern- und Erinnerungseinschränkungen, Schlafstörungen, entzündungsbedingte Hyperalgesie, Hirnödemen, Unterversorgungszuständen (Hypoxie), entzündliche Darmerkrankungen und der damit verbundenen Krankheitssymptome, funktionelle Magen-Darm-Erkrankungen, die mit Schmerzen und/oder einer vermehrten oder verminderten Peristaltik einhergehen, insbesondere des Irritable bowel Syndroms, zur Behandlung und/oder Prophylaxe der nicht mit einem Ulkus verbundenen Dyspepsie, Obstipation, insbesondere opiod-induzierte Obstipation, Arthritis, Migräne, Psoriasis oder anderen juckenden Hauterkrankungen, Dysmenorrhoe, Fibromylagia, Schmerzzuständen, insbesondere von Schmerzüberempfindlichkeitsreaktionen auftretend bei Rückenleiden, Brandverletzungen, Sonnenbrand und rheumatischen Erkrankungen, postoperativen Schmerzen sowie des häufig nach Abdominaloperationen auftretenden Ileus, Erkrankungen der Blase, insbesondere der Reizblase, auch irritable bladder, Zytalgie, Zystalgie, Neuralgia oder Blasenneurose.

Die Verbindungen der Formel I und/oder deren Enantiomere, Diastereomere, Racemate sowie deren physiologisch verträglichen Salze und Solvate können als Arzneimittelwirkstoff in der Human- und Veterinärmedizin eingesetzt werden.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können vorzugsweise erhalten werden, indem man Verbindungen der Formel II mit Verbindungen der Formel III, vorzugsweise mit Verbindungen der Formel IIIa oder Verbindungen der Formel IV mit Verbindungen der Formel V umsetzt.

Gegenstand der Erfindung ist daher ein Verfahren zur Herstellung der Verbindungen der Formel I und deren physiologisch verträglichen Salze und Solvate, dadurch gekennzeichnet, dass man
a) eine Verbindung der Formel II 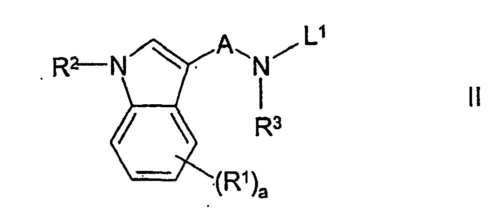 worin L¹ H oder ein Metallion bedeutet und R¹, R², R³, A und a die vor- und nachstehend für die Verbindungen der Formel I angegebenen Bedeutungen haben,
b) mit einer Verbindung der Formel III, 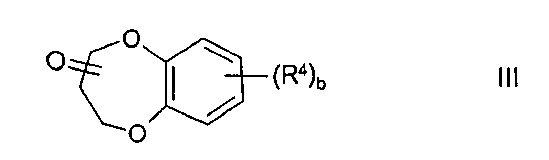 vorzugsweise mit einer Verbindung der Formel IIIa, 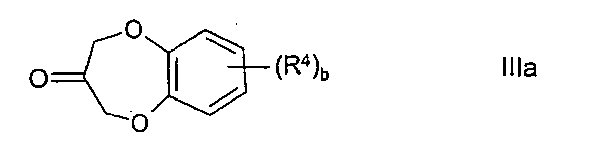 wobei in Formel III und IIIa R⁴ und b die vor- und nachstehend für die Verbindungen der Formel I angegebenen Bedeutungen haben, umsetzt,
c) gegebenenfalls einen Reduktionsschritt durchführt und
d) gegebenenfalls die erhaltene Verbindung der Formel I durch Behandeln mit einer Säure in eines ihrer Salze überführt.

Gegenstand der Erfindung ist weiterhin ein Verfahren zur Herstellung der Verbindungen der Formel I und deren physiologisch verträglichen Salze und Solvate, dadurch gekennzeichnet, dass man
a) eine Verbindung der Formel IV 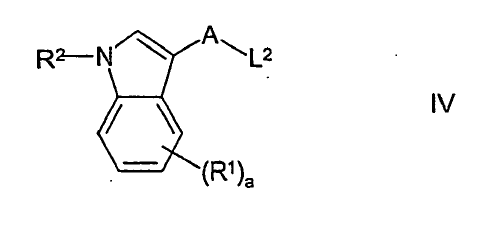 worin L² Cl, Br, I, OH, eine reaktionsfähig veresterte OH-Gruppe oder eine Diazoniumgruppe bedeutet und R¹, R², A und a die vor- und nachstehend für die Verbindungen der Formel I angegebenen Bedeutungen haben,
b) mit einer Verbindung der Formel V, 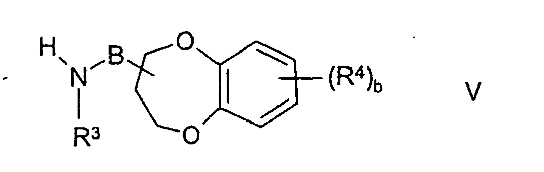 worin R³, R⁴, B und b die vor- und nachstehend für die Verbindungen der Formel I angegebenen Bedeutungen haben,
   umsetzt, und gegebenenfalls
c) die erhaltene Verbindung der Formel I durch Behandeln mit einer Säure in eines ihrer Salze überführt.

Die erfindungsgemäßen Verfahren können im Sinne einer Eintopfreaktion durchgeführt werden, d. h. auf Isolierungs- und/oder Reinigungsschritte wird so weit wie möglich verzichtet und nur das gewünschte Endprodukt wird gereinigt und/oder isoliert. Alternativ kann nach jedem der genannten Reaktionsschritte ein Reinigungs- und/oder Isolierungsschritt durchgeführt werden. Auch gemischte Formen der vorstehend beschriebenen Verfahrensweisen sind denkbar.

Geeignete Reinigungs- und Isolierungsschritte sind dem Fachmann bekannt, z. B. aus Houben-Weyl, Methoden der organischen Chemie, Georg-Thieme-Verlag, Stuttgart.

Die Ausgangsstoffe, z. B. die Verbindungen der Formel II, III, IIIa, IV oder V können, falls erwünscht, auch in situ gebildet werden, so dass man sie aus dem Reaktionsgemisch nicht isoliert, sondern sofort weiter zu der Verbindung der Formel I umsetzt.

Die Umsetzung sowohl der Verbindungen der Formel II mit den Verbindungen der Formel III, bevorzugterweise mit den Verbindungen der Formel IIIa als auch die Umsetzung der Verbindungen der Formel IV mit den Verbindungen der Formel V erfolgt in der Regel in einem inerten Lösungsmittel.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Die Umsetzung der Verbindungen der Formel IV mit Verbindungen der Formel V erfolgt in der Regel in Gegenwart eines säurebindenden Mittels. Als säurebindende Mittel kommen alle in der organischen Synthesechemie üblichen Basen, sowohl anorganische als auch organische, bevorzugt organische Basen, in Betracht. Beispiele für geeignete organische Basen sind Triethylamin, Diisopropylamin (DIPEA), Dimethylanilin, Pyridin oder Chinolin. Auch der Zusatz einer anorganischen Base, wie beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, kann günstig sein.

Die Umsetzung der Verbindungen der Formel II mit den Verbindungen der Formel III oder IIIa erfolgt in der Regel ebenfalls in einem der oben genannten inerten Lösungsmittel. Ebenfalls kann es zweckmäßig sein, anschließend an die Umsetzung der Verbindung der Formel II und III beziehungsweise IIIa einen Reduktionsschritt durchzuführen. Geeignete Reduktionsschritte sind dem Fachmann bekannt. Vorzugsweise kann der Reduktionsschritt durch Reduktion mit Metallhydriden, beispielsweise komplexen Metallhydriden, durchgeführt werden. Beispiele für Metallhydride sind Natriumhydrid, Kalziumhydrid, Natriumboranat und Lithiumaluminiumhydrid, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AICI₃ oder LiBr. Bevorzugt ist die Reduktion mit komplexen Metallhydriden, wie NaBH₄ und LiAlH₄. besonders bevorzugt ist die Reduktion mit NaBH₄. Als Lösungsmittel eignen sich hierfür insbesondere Ether, wie Diethylether, Din-butylether, THF, Dioxan, Diglyme oder 1,2-Dimethoxyethan, sowie Kohlenwasserstoffe, wie Benzol. Für eine Reduktion mit NaBH₄ sind in erster Linie Alkohole wie Methanol oder Ethanol, ferner Wasser sowie wässrige Alkohole als Lösungsmittel geeignet.

Die Reaktionszeit liegt je nach den angewendeten Bedingungen zwischen einigen Minuten und 14 Tagen, die Reaktionstemperatur zwischen etwa -30 °C und 180 °C, normalerweise zwischen -20 °C und 140 °C, bevorzugt zwischen -10 °C und 130 °C und insbesondere zwischen etwa 0 °C und etwa 120 °C.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht verträglichen Säuren, z.B. Pikrate, können zur Isolierung und/oder Aufreinigung der Verbindungen der Formel I verwendet werden.

Gegenstand der Erfindung sind insbesondere diejenigen Verbindungen der Formel I, in denen mindestens einer der genannten Reste eine der vor- oder nachstehend angegebenen bevorzugten Bedeutungen hat.

Im Rahmen der vorliegenden Erfindung bedeutet Alkyl einen linearen oder verzweigten Alkylrest, vorzugsweise einen unverzweigten Alkylrest, der 1, 2, 3, 4 oder 5 C-Atome, vorzugsweise 1, 2, oder 3 C-Atome aufweist und ein- oder mehrfach mit Halogen (Hal), z. B. perfluoriert, sein kann. Wenn ein Alkylrest mit Halogen substituiert ist, weist er vorzugsweise, abhängig von der Anzahl der Kohlenstoffatome des Alkylrests, 1, 2, 3, 4 oder 5 Halogenatome auf. So kann beispielsweise eine Methylgruppe (Alkylrest mit 1 Kohlenstoffatom) 1-, 2- oder 3-fach mit Halogen substituiert sein, und eine Ethylgruppe (Alkylrest mit 2 Kohlenstoffatomen) 1-, 2-, 3-, 4- oder 5-fach mit Halogen substituiert sein.
Für Alkylgruppen mit mehr als 2 Kohlenstoffatomen gilt vorzugsweise das gleiche wie für Ethylgruppen. Besonders bevorzugt steht Alkyl für Methyl, Ethyl, Trifluormethyl, Pentafluorethyl oder Propyl, weiterhin bevorzugt für Isopropyl, Butyl, Isobutyl, sek.-Butyl oder tert.-Butyl, aber auch für n-Pentyl, neo-Pentyl oder Isopentyl.

Der Ausdruck Alkoxy um fasst die Reste -O-Alkyl, wobei Alkyl die oben genannte Bedeutung hat. Bevorzugt sind die Reste Methoxy, Ethoxy und Propoxy.

Der Ausdruck "Alkoxyalkyl" umfaßt vorzugsweise geradkettige Reste der Formel C_{U}H_{2U+1}-O-(CH₂)ᵥ-, worin u und v jeweils unabhängig voneinander 1, 2, 3 oder 4 bedeuten, wobei die Summe aus u und v 5 jedoch nicht überschreitet. Besonders bevorzugt ist u = 1 und v= 1, 2, 3 oder 4. Der Ausdruck "Aryl" umfasst vorzugsweise einen unsubstituierten oder ein- oder mehrfach substituierten Benzolring, z.B. einen unsubstituierten oder substituierten Phenylrest oder ein unsubstituiertes oder ein- oder mehrfach substituiertes System aus Benzolringen, wie zum Beispiel Anthracen-, Phenanthren- oder Napthalen-Ringsysteme. Beispiele für geeignete Substituenten umfassen Alkyl-, Alkoxy-, Oxo-, Hydroxy-, Mercapto-, Amino-, Nitro-, Cyano- und Halogen-Reste.

Der Ausdruck "Aralkyl" umfasst vorzugsweise einen Arylrest wie obenstehend definiert, verbunden mit einem Alkylrest wie obenstehend definiert. Beispiele für geeignete Aralkylreste umfassen, sind aber nicht beschränkt auf, Benzyl, Phenylpropyl, Phenylbutyl und dergleichen.

Der Ausdruck "Heteroalkyl" umfasst vorzugsweise einen Alkylrest wie obenstehend definiert, in dem ein oder mehrere Kohlenstoffatome durch mindestens ein Sauerstoff-, Stickstoff- oder Schwefelatom ersetzt sind, z. B. eine Alkyloxy-Gruppe wie z. B. Kethoxy oder Ethoxy, oder eine Methoxymethyl-, Cyano- oder 2,3-Dioxyethyl-Gruppe, Polyoxyethylen- oder -propylenreste oder Polythioethylen- oder propylenreste.

In den Verbindungen der Formel II bedeutet L¹ vorzugsweise H oder eine die Aminofunktion aktivierende Gruppe, beispielsweise ein Metallion. In einer besonders bevorzugten Ausführungsform bedeutet L¹ H. Geeignete Metallionen sind insbesondere Alkalimetall-, Erdalkalimetall- oder Aluminium-Ionen. Bevorzugt als Metallionen sind Alkalimetallionen, insbesondere Li, Na oder K. Bei mehrwertigen Metallionen bildet sich oft ein Komplex aus Metallion und zwei oder mehreren Verbindungen der Formel III, wobei der Komplex stöchiometrisch in der Regel so viele Verbindungen der Formel III umfasst, wie es der Wertigkeit des Metallion entspricht.

In den Verbindungen der Formel IV bedeutet L² vorzugsweise Cl, Br, I, OH, eine reaktionsfähig abgewandelte OH-Gruppe, insbesondere eine reaktionsfähig veresterte OH-Gruppe, wie eine Alkylsulfonyloxy-Gruppe mit 1-6 C-Atomen (bevorzugt Methylsulfonyloxy) oder Arylsulfonyloxy-Gruppe mit 6-10 C-Atomen (bevorzugt Phenyl- oder p-Tolylsulfonyloxy), oder eine Diazoniumgruppe. In einer besonders bevorzugten Ausführungsform bedeutet L² Cl.

Der Ausdruck Solvate im Sinne der vorliegenden Erfindung umfasst einen Komplex variabler Stöchiometrie aus einer gelösten Verbindung nach Formel 1 oder einem Salz davon und einem in Bezug auf die biologische Aktivität der Verbindung nach Formel I inerten Lösungsmittel. Beispiele für geeignete Lösungsmittel umfassen zum Beispiel Wasser, Methanol, Ethanol oder Essigsäure.

Gegenstand der vorliegenden Erfindung sind bevorzugt Verbindungen der Formel I, worin die Reste R² und R³ für H stehen, wobei R¹, R⁴, R⁵, A, B, D, a, b und Hal die vor- und nachstehend für die Verbindungen der Formel I angegebenen Bedeutungen haben.

Weiterhin sind Verbindungen der Formel I bevorzugt, worin die Reste R² und R³ für H und mindestens ein Rest R¹ für (CH₂)ₘCN steht, wobei R⁴, R⁵, A, B, D, a, b, m und Hal die vor- und nachstehend für die Verbindungen der Formel I angegebenen Bedeutungen haben. Unter diesen Verbindungen sind die diejenigen Verbindungen, bei denen R¹ in 5-Stellung des Indolringes und a vorzugsweise für 1 und m vorzugsweise für 0 steht, besonders bevorzugt.

Weitere bevorzugte Verbindungen der Formel I sind solche worin A für CₙH₂ₙ mit n = 4 und B für CₚH₂ₚ mit p = 1 oder 0 steht, wobei R¹, R² R³, R⁴, R⁵, D, a, b, m und Hal die vor- und nachstehend für die Verbindungen der Formel I angegebenen Bedeutungen haben.

Besonders bevorzugte Verbindungen der Formel I sind solche worin R¹ für (CH₂)ₘCN in 5-Stellung des Indolringes und a für 1 und m für 0, R² und R³ für H, A für CₙH₂ₙ mit n = 4 und B für CₚH₂ₚ mit p = 1 oder 0 steht, wobei, R⁴, R⁵, D, und Hal die vor- und nachstehend für die Verbindungen der Formel I angegebenen Bedeutungen haben.

Die vorstehend als bevorzugt genannten Reste sind auch in den Verbindungen der Formeln II, III, IIIa, IV und V bevorzugt.

In einer ganz besonders bevorzugten Ausführungsform der vorliegenden Erfindung sind die Verbindungen der Formel I ausgewählt unter den folgenden Teilformeln I a bis I e sowie deren physiologisch verträglichen Salzen und Solvaten.

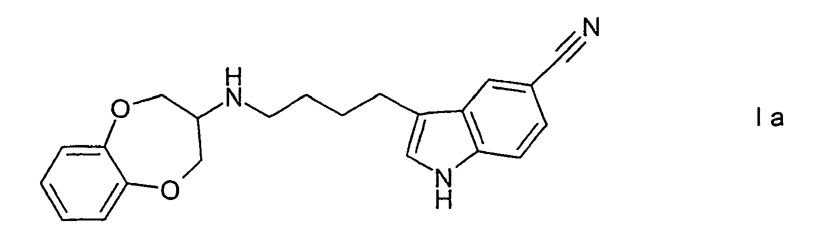

### N-(3,4-Dihydro-2H-1,5-benzodioxepin-3-yl)-4-(5-cyano-3-indolyl)butylamin

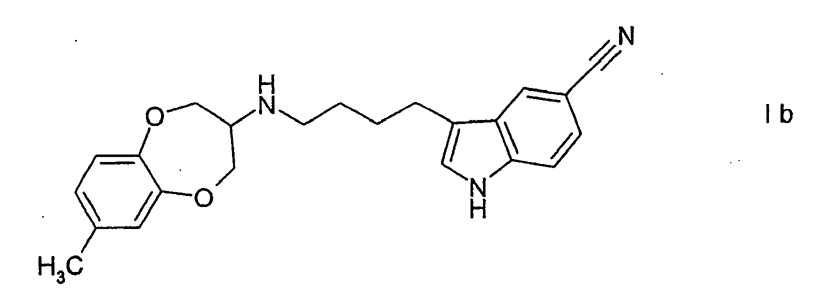

### 3-{4-[7-Methyl-3,4-dihydro-2H-1,5-benzodioxepin-3-ylamino]-butyl}-indol-5-carbonitril

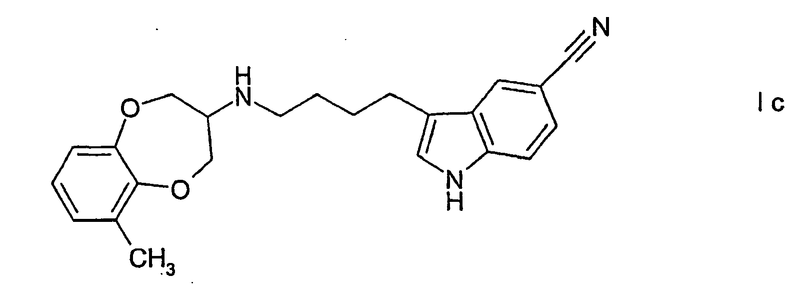

### 3-{4-(6-Methyl-3,4-dihydro-2H-1,5-benzodioxepin-3-ylamino)-butyl}-indol-5-carbonitril

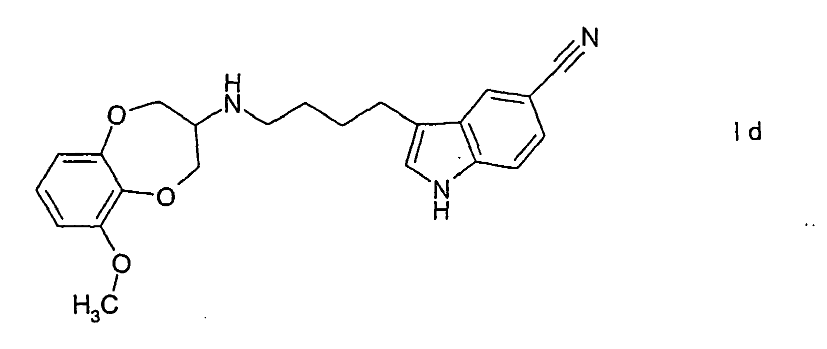

### 3-[4-(6-Methoxy-3,4-dihydro-2H-1,5-benzodioxepin-3-ylamino)-butyl]-indol-5-carbonitril

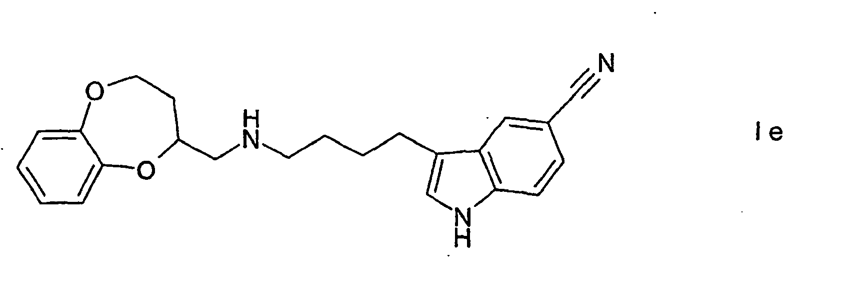

### 3-[4-(3,4-Dihydro-2H-1,5-benzodioxepin-3-yl)-methylamino)-butyl]-indol-5-carbonitril

Die erfindungsgemäßen Verbindungen nach Formel I können, abhängig von der Auswahl der vorstehend beschriebenen Substituenten und Reste, ein oder mehrere chirale Zentren, insbesondere ein oder mehrere chirale Kohlenstoffatome, aufweisen. Wenn eine erfindungsgemäße Verbindung definierter Zusammensetzung ein oder mehrere chirale Zentren aufweist, kann diese Verbindung definierter Zusammensetzung in unterschiedlichen Stereoisomeren vorliegen. Gegenstand der vorliegenden Erfindung sind alle möglichen solchen Stereoisomere erfindungsgemäßer Verbindungen, die sowohl als einzelne, stereochemisch einheitliche Verbindungen, als auch als Gemische zweier oder mehrerer stereochemisch einheitlicher Verbindungen vorliegen können. Im Falle von Gemischen zweier oder mehrerer Stereoisomere können die einzelnen Stereoisomere in unterschiedlichen oder gleichen Anteilen vorliegen. Bei Gemischen aus zwei Stereoisomeren, die in gleichen Anteilen vorliegen und optische Antipoden darstellen, spricht man von racemischen Gemischen. Racemische Gemische von Verbindungen der Formel I sind ebenfalls Gegenstand der vorliegenden Erfindung.

Die Erfindungsgemäßen Verbindungen können als Therapeutika, Diagnostika und/oder Kosmetika beziehungsweise zusammen mit einem oder mehreren von den erfindungsgemäßen Verbindungen verschiedenen Wirkstoffen und/oder Hilfsstoffen in Therapeutika, Diagnostika und oder Kosmetika verwendet werden. Üblicherweise werden die erfindungsgemäßen Verbindungen in Form von pharmazeutischen, diagnostischen und/oder kosmetischen Formulierungen eingesetzt. Solche Formulierungen und Verfahren zu ihrer Herstellung sind dem Fachmann bekannt.

Beispiele für solche Formulierungen sind wenigstens eine erfindungsgemäße Verbindung enthaltende Suspensionen, Emulsionen, Lösungen, Liposome, Salze, Pasten, bioabbaubare Polymere, Nanopartikel, Tabletten, beschichtete Tabletten, Dragees, Filmtabletten, Kapseln, Pillen, Granulate, Pulver, Aerosole, Tropfen oder Sprays.

Die erfindungsgemäßen Verbindungen bzw. Formulierungen, die wenigstens eine erfindungsgemäße Verbindung enthalten, können an Mensch oder Tier verabreicht werden, z. B. lokal oder systemisch und insbesondere oral, intravenös, intraperitoneal, subkutan, transdermal, nasal, buccal und/oder ionotophoretisch.

Gegenstand der Erfindung ist ferner die Verwendung der Verbindungen der Formel I und/oder ihrer physiologisch verträglichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend eine wirksame Menge mindestens einer der Verbindungen der Formel I und/oder eines ihrer physiologisch verträglichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.

Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Gegenstand der vorliegenden Erfindung sind daher auch Verfahren zur Herstellung pharmazeutischer Zubereitungen, die dadurch gekennzeichnet sind, dass man eine Verbindung der Formel I und/oder eines ihrer physiologischen verträglichen Salze und/oder eines ihrer Solvate zusammen mit mindestens einem festen, flüssigen oder halbflüssigen Träger- oder Hilfsstoff in eine geeignete Dosierungsform bringt.

Dabei können die erfindungsgemäßen Verbindungen in der Regel in Analogie zu anderen bekannten Verbindungen mit ähnlichem Wirkprofil verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Die Wirksamkeit der erfindungsgemäßen Verbindungen für die Behandlung von Erkrankungen der Blase, insbesondere der Reizblase, kann mit üblichen, aus dem Stand der Technik bekannten Methoden nachgewiesen werden, beispielsweise mit den im Folgenden beschriebenen Tiermodellen, oder in dazu analoger Weise.

Ein Modell zur Messung des Einflusses auf die Urinausscheidung ist bei Lipschitz et. al., J. Pharmacol. Exp. Ther. 1943; 79: 97-110 beschrieben. Die zu untersuchende Substanz wird Ratten gegeben, denen vorher über Nacht das Futter entzogen wurde bei freiem Wasserzugang. Eine erhöhte Urinauscheidung wird provoziert durch die gleichzeitige intraperitonelae Injektion von 100 ml/kg physiologischer Kochsalzlösung. Unmittelbar nach Substanzgabe wurde die Harnblase durch leichtes Massieren des Abdomens über der Harnblase entleert. Anschließend werden die Ratten in Metabolismuskäfigen gehalten, in denen der Urin über den Zeitraum von 6 Stunden aufgefangen wird. Die erfindungsgemäßen Verbindungen erhöhen vorzugsweise die Urinausscheidung dosisabhängig, wobei beispielsweise bei einer Dosis von etwa 100 mg/kg die Ausscheidung einer stark erhöhten Urinmenge, vorzugsweise wenigstens einer 2fach höheren Urinmenge, beobachtet werden kann. In Analogie kann die Wirkung auf die Urinausscheidung bei normalen Ratten geprüft (d.h. ohne die Induktion einer erhöhten Urinausscheidung, s.o.) werden. Auch hier erhöhen die erfindungsgemäßen Verbindungen vorzugsweise die Urinausscheidung dosisabhängig, wobei hier in vielen Fällen bereits bei geringeren Dosen der erfindugsgemäßen Verbindungen, beispielsweise bei etwa 30 mg/kg po, die Ausscheidung einer höheren Urinmenge, beispielsweise einer 5fach höheren Urinmenge, beobachtet werden kann.

Das klassische Tiermodell für die Reizblase ist beschrieben bei Ghoniem et el., Neurourol. Urodyn. 1995; 14: 657-65. Bei weiblichen Affen wird eine Reizblase durch die direkte Infusion von Aceton in die Blase induziert. Die Tiere werden in Metabolismuskäfigen gehalten, die für das kontinuierliche Monitoring der Miktion (Hamlassen) der Tiere ausgelegt sind. Über Harnflussmesser werden Frequenz, Entleerungsvolumen und Flussgeschwindigkeit des Hams kontinuierlich gemessen. Der Vergleich der Harnstoffabsorption vor und nach der Acetoninfusion zeigt, dass die Hamstoffabsorption nach Acetoninfusion drastisch erhöht ist und erst nach vier Wochen wieder den Basiswert vor der Acetoninfusion erreicht. Ferner werden in der ersten Woche nach Acetoninfusion starke Veränderungen der Blasenphysiologie beobachtet: Die Blasenleistung, gemessen in ml/cm, erniedrigt sich um fast 35%. Auch das Entleerungsverhalten ändert sich stark, wobei die Frequenz der Entleerungen stark ansteigt mit dem Bild eines häufigen Tröpfelns und dabei mit einem um ca. 70% verminderten Entleerungsvolumen. Die systematische Verhaltensbeobachtung der Tiere über vier Wochen ergibt als Verhaltensrepertoire eine erniedrigte Frequenz allgemeiner und insbesondere sozialer Aktivitäten, während stereotype, auf sich selbst bezogene Verhaltensweisen wie sich Lausen, Kratzen, Kraulen stark zunehmen. Diese bei den Affen beobachteten Verhaltensänderungen sind konsistent mit dem klinischen Bild erheblicher Beschwerden und Schmerzen. Bei Verabreichung die erfindungsgemäßen Verbindungen in üblichen Dosierungen und insbesondere wie vorstehend beschriebenen Dosierungen, wie beispielsweise Dosierungen von 3, 10, und 30 mg/kg, kann vorzugsweise eine dosisabhängige Normalisierung der Blasenfunktion beobachtet werden.

### Beispiel 1

### Synthese von N-(3,4-Dihydro-2H-1,5-benzodioxepin-3-yl)-4-(5-cyano-3-indol)butylamin (= EMD 76066)

Die Synthese des hier verwendeten Ausgangsmaterials ist literaturbekannt (Sci. Pharm. 201, 69(1), 11-20).
Es werden 4 g (24 mmol) Benzo[b][1,4]dioxepin-3-on und 5,2 g (24 mmol) 3-(4-Aminobutyl)-1 H-5-indolcarbonitril in 270 ml Methanol gelöst und für 2h zum Rückfluss erhitzt. Nachdem der Ansatz auf 10 °C abgekühlt worden ist, gibt man 1,4 g (36 mmol) Natriumborhydrid portionsweise hinzu und erhitzt nach einer Stunde für eine Stunde zum Rückfluss. Der Ansatz wird zur Trockene eingeengt und in Essisäurethylester aufgenommen. Nach dem Waschen mit Wasser und Trocknen mit Magnesiumsulfat wird der Rückstand mit einem 7:3 Gemisch aus Essigsäureethylester und Isopropanol über eine Kieselgelsäule chromatographiert. Man erhält 1,86 g (16 %) N-(3,4-Dihydro-2H-1,5-benzodioxepin-3-yl)-4-(5-cyano-3-indol)butylamin.
Smp.: 166-168 °C
CHN berechnet: C: 65,39 H: 5,70 N: 8,80
CHN gefunden: C: 65,39 H: 5,82 N: 8,77
[M+H]⁺, (ESI-MS): 362

### Beispiel 2

### Synthese von 3-[4-(3,4-Dihydro-2H-1,5-benzodioxepin-3-yl)-methylamino)-butyl]-indol-5-carbonitril (= EMD 87322)

Die Synthese des hier verwendeten Ausgangsmaterials ist literaturbekannt (J. Med. Chem. 1984, 27, 570).
a) Bei Raumtemperatur versetzt man eine Suspension von 5,2 g (27 mmol) 3,4-Dihydro-2H-1,5-benzodioxepin-2-yl)methylamid langsam mit 22,5 ml(81 mmol) RedAl^{®}, was zu einer Temperaturerhöhung auf 30 °C führt. Die resultierende Lösung lässt man über 2 h auf Raumtemperatur abkühlen. Man gibt nachfolgend 100 ml Wasser tropfenweise hinzu und extrahiert drei mal mit 30 ml Essigsäureethylester. Die vereinigten organischen Phasen werden mit Natriumsulfat getrocknet und nach abfiltrieren des Salzes eingeengt. Das resultierende Öl (4,3 g; 83 %) ist so sauber, dass es ohne weitere Reinigung weiter umgesetzt werden kann.
b) In 100 ml Acetonitril löst man 4,3 g (24 mmol) 4-(3,4-Dihydro-2H-1,5-benzobenzodioxepin-3-yl)methylaminund erhitzt die Lösung für eine Stunde zum Rückfluss. Nach dem Abkühlen gibt man eine Lösung von 5 g 3-(4-Chlorbutyl)-indol-5-carbonitril hinzu und erhitzt für eweitere 18 h zum Rückfluss (DC-Kontrolle). Nach dem Abkühlen der Reaktionslösung wird diese zum Rückstand eingeengt, mit 250 ml Wasser verrührt und drei mal mit 50 ml Diethylether extrahiert. Nach dem Trocknen der organichen Phase über Natriumsulfat wird das Lösungsmittel am Rotationsverdampfer entfernt und die resultierenden 9,2 g Rohsubstanz über eine Kieselgel-Säule gereinigt. Die Produktfraktion wurde nach dem Eindampfen aus Essigsäureethylester und Pentan gefällt und die resultierenden Kristalle nachfolgend aus Diisopropylether und Essigsäureethylester umkristallisiert. Man erhält 0,3 g (4 %) des Produkts als Monohydrat.
   Smp.: 78 °C
   [M+H]⁺, (ESI-MS): 376

Analog der Beispiele 1 und 2 erhält man die Verbindungen:
3-{4-[7-Methyl-3,4-dihydro-2H-1,5-benzodioxepin-3-ylamino]-butyl}-indol-5-carbonitril (EMD 85350): [M+H]⁺, (ESI-MS): 376
3-{4-(6-Methyl-3,4-dihydro-2H-1,5-benzodioxepin-3-ylamino)-butyl}-indol-5-carbonitril (EMD 87319); [M+H]⁺, (ESI-MS): 376
3-[4-(6-Methoxy-3,4-dihydro-2H-1,5-benzodioxepin-3-ylamino)-butyl]-indol-5-carbonitril (EMD 87326); [M+H]⁺, (ESI-MS): 392

### Beispiel 3

Die Wirksamkeit der Verbindungen nach Formel I wird mit folgenden Untersuchungen überprüft:

Die Serotoninwiederaufnahmehemmung wurde mit Hilfe der synaptosomalen Aufnahmehemmung nach Wong et al. (Neuropsychopharmacol. 8 (1993), 23- 33) untersucht.

Die Bindungseigenschaften zu dem 5HT1A Rezeptor wurde mit Hilfe des 5-HT1A-(Serotonin)-Bindungstests bestimmt (Matzen et al., J. Med. Chem., 43 (2000), 1149-1157, insbesondere Seite 1156 mit Verweis auf Eur. J. Pharmacol.: 140 (1987), 143-155).

Zur Bestimmung der Bindungseigenschaften an den 5-HT4 Rezeptor wurde der Test nach Grossman et al. herangezogen (Grossman et al., Br. J. Pharmacol. 109, (1993), 618-24).

Dabei wurden folgende Werte gefunden:

| Verbindung | 5HT1A (IC₅₀ in nmol/l) | 5HT4 (IC₅₀ in nmol/l) | SSRI (IC₅₀ in nmol/l) |
|---|---|---|---|
| EMD 76066 | 0,5 | n.d. | 8,0 |
| EMD 87322 | 4,0 | 8,4 | 0,6 |
| EMD 85350 | 3,0 | n.d. | 4,0 |
| EMD 87319 | 2,0 | n.d. | 3,0 |
| EMD 87326 | 0,6 | n.d. | 1,0 |

| | | | |
|---|---|---|---|
| n.d. = nicht durchgeführt | | | |

Die nachfolgenden Beispiele betreffen pharmazeutische Zubereitungen:

### Beispiel A: Injektionsgläser

Eine Lösung von 100 g des Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g des Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g des Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg des Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepresst, die anschließend in üblicher Weise mit einem Überzug aus Saccharose, Kartoflelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Benzdioxepine der Formel I 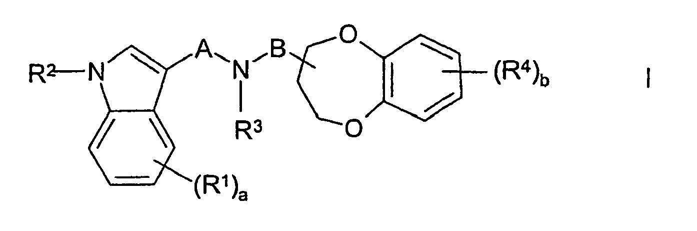 worin
R¹ unabhängig voneinander ausgewählt ist unter Alkyl, (CH₂)ₘOD, (CH₂)ₘCN, (CH₂)ₘCOR⁵ oder (CH₂)ₘCH₂R⁵ wobei m=0 oder 1 ist,
R², R³ unabhängig voneinander ausgewählt ist unter H, Alkyl mit 1 bis 5 C-Atomen,
R⁴ unabhägig voneinander ausgewählt ist unter Alkyl mit 1 bis 5 C-Atomen, Heteroalkyl mit 1 bis 5 C-Atomen, Alkoxy mit 1 bis 5 C-Atomen, Alkoxyalkyl mit 2 bis 5 C-Atomen, Hal, CN, COR⁵ oder OH,
R⁵ für OD, NH₂, NHD oder ND₂,
A für CₙH₂ₙ mit n= 2, 3, oder 4,
B für CₚH₂ₚ mit p=0, 1, 2, 3 oder 4 steht,
D unabhängig voneinander ausgewählt ist unter H, Alkyl mit 1 bis 5 C-Atomen, Alkoxyalkyl mit 2 bis 5 C-Atomen, Aryl oder ArAlkyl
a, b für 0, 1 oder 2 und
Hal für F, Cl, Br oder I steht
sowie deren physiologisch verträgliche Salze und Solvate.

2. Benzdioxepine der Formel I gemäss Anspruch 1, **dadurch gekennzeichnet, dass** die Reste R² und R³ für H stehen.

3. Benzdioxepine der Formel I gemäss Anspruch 1 oder 2, **dadurch gekennzeichnet, dass** die Reste R² und R³ für H und mindestens ein Rest R¹ für (CH₂)ₘCN steht.

4. Benzdioxepine der Formel I gemäss Anspruch 1, 2 oder 3, **dadurch gekennzeichnet, dass** ein (CH₂)ₘCN in 5-Stellung des Indolringes und a vorzugsweise für 1 und m vorzugsweise für 0 steht.

5. Benzdioxepine der Formel I gemäss einem der vorhergehenden Ansprüche, **dadurch gekennzeichnet, dass** A für CₙH₂ₙ mit n = 4 und B für CₚH₂ₚ mit p = 1 oder 0 steht.

6. Benzdioxepine der Formel I gemäss einem der vorhergehenden Ansprüche, **dadurch gekennzeichnet, dass** R¹ für (CH₂)ₘCN in 5-Stellung des Indolringes und a für 1 und m für 0, R² und R³ für H, A für CₙH₂ₙ mit n = 4 und B für CₚH₂ₚ mit p = 1 oder 0 steht.

7. Benzdioxepine der Formel I gemäss einem der vorhergehenden Ansprüche ausgewählt aus der Gruppe bestehend aus
N-(3,4-Dihydro-2H-1,5-benzodioxepin-3-yl)-4-(5-cyano-3-indolyl)butylamin,
3-{4-[7-Methyl-3,4-dihydro-2H-1,5-benzodioxepin-3-ylamino]-butyl}-indol-5-carbonitril,
3-{4-(6-Methyl-3,4-dihydro-2H-1,5-benzodioxepin-3-ylamino)-butyl}-indol-5-carbonitril,
3-[4-(6-Methoxy-3,4-dihydro-2H-1,5-benzodioxepin-3-ylamino)-butyl]-indol-5-carbonitril und
3-[4-(3,4-Dihydro-2H-1,5-benzodioxepin-3-yl)-methylamino)-butyl]-indol-5-carbonitril.

8. Verfahren zur Herstellung von Benzdioxepinen der Formel 1 nach einem der Ansprüche 1 bis 7, **dadurch gekennzeichnet, dass** man
a) eine Verbindung der Formel II 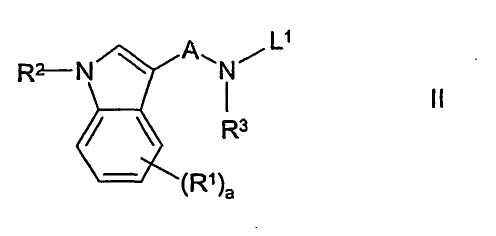 worin L¹ H oder ein Metallion bedeutet und R¹, R², R³, A und a die die in Anspruch 1 angegebenen Bedeutungen haben,
b) mit einer Verbindung der Formel III, 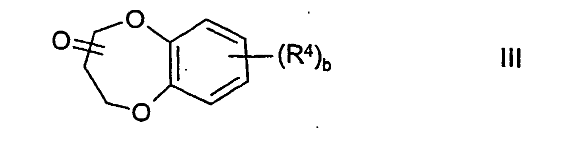 wobei in Formel III R⁴ und b die vor- und nachstehend für die in Anspruch 1 angegebenen Bedeutungen haben, umsetzt und gegebenenfalls
c) gegebenenfalls einen Reduktionsschritt durchführt und
d) gegebenenfalls die erhaltene Verbindung der Formel I durch Behandeln mit einer Säure in eines ihrer Salze überführt.

9. Verfahren zur Herstellung von Benzdioxepinen der Formel 1 nach einem der Ansprüche 1 bis 7 sowie deren physiologisch verträgliche Salze und Solvate, **dadurch gekennzeichnet, dass** man
a) eine Verbindung der Formel IV 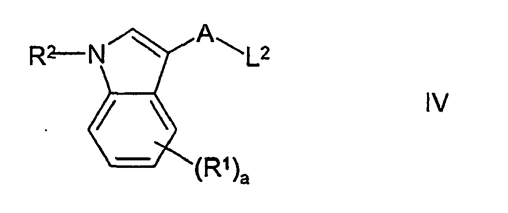 worin L² Cl, Br, I, OH, eine reaktionsfähig veresterte OH-Gruppe oder eine Diazoniumgruppe bedeutet und R¹, R², A und a die in Anspruch 1 angegebenen Bedeutungen haben,
b) mit einer Verbindung der Formel V, 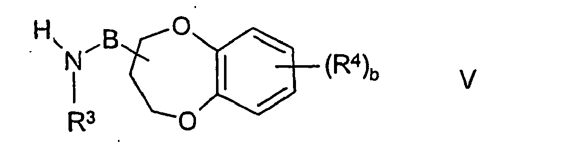 worin R³, R⁴, B und b die in Anspruch 1 angegebenen Bedeutungen haben, umsetzt, und gegebenenfalls
c) die erhaltene Verbindung der Formel I durch Behandeln mit einer Säure in eines ihrer Salze überführt.

10. Verbindungen der Formel 1 nach einem der Ansprüche 1 bis 7 und/oder deren physiologisch verträgliche Salze und Solvate als Arzneimittel.

11. Verwendung der Verbindungen der Formel 1 nach einem der Ansprüche 1 bis 7 zur Herstellung eines Arzneimittels zur Behandlung und/oder Prophylaxe verschiedener Erkrankungen wie z.B. Schlaganfall, cerebrale Ischämie sowie Minderung von Folgeschäden einer Ischämie, Traumata, Hypoglykämie, Schizophrenie, Depression, Demenz, Dyskinesie, neurodegenerative Erkrankungen wie Parkinsonsche Krankheit, ALS, Morbus Alzheimer, Lewy bodies Dementia oder Huntington Syndrom, Tourette Syndrom, Angst, Lern- und Erinnerungseinschränkungen, Schlafstörungen, entzündungsbedingte Hyperalgesie, Himödemen, Unterversörgungs-zuständen (Hypoxie), entzündliche Darmerkrankungen und der damit verbundenen Krankheitssymptome, funktionelle Magen-DarmErkrankungen, die mit Schmerzen und/oder einer vermehrten oder verminderten Peristaltik einhergehen, insbesondere des Irritable bowel Syndroms, zur Behandlung und/oder Prophylaxe der nicht mit einem Ulkus verbundenen Dyspepsie, Obstipation, insbesondere opiod-induzierte Obstipation, Arthritis, Migräne, Psoriasis oder anderen juckenden Hauterkrankungen, Dysmenorrhoe, Fibromylagia, Schmerzzuständen, insbesondere von Schmerzüberempfindlichkeitsreaktionen auftretend bei Rückenleiden, Brandverletzungen, Sonnenbrand und rheumatischen Erkrankungen, postoperativen Schmerzen sowie des häufig nach Abdominaloperationen auftretenden Ileus, Erkrankungen der Blase, insbesondere der Reizblase, auch irritable bladder, Zytalgie, Zystalgie, Neuralgia oder Blasenneurose.

12. Verfahren zur Herstellung einer pharmazeutischen Zubereitung, **dadurch gekennzeichnet, dass** man mindestens eine Verbindung der Formel I nach einem der Ansprüche 1 bis 7 und/oder eines ihrer physiologisch verträglichen Salze oder Solvate zusammen mit mindestens einem festen, flüssigen oder halbfesten Träger- oder Hilfsstoff in eine geeignete Dosierungsform bringt.

13. Pharmazeutische Zubereitung, **dadurch gekennzeichnet, dass** sie einen wirksamen Gehalt an mindestens einer Verbindung der Formel I nach einem der Ansprüche 1 bis 7 und/oder eines ihrer physiologisch verträglichen Salze oder Solvate enthält.

## Claims

1. Benzodioxepines of the formula I 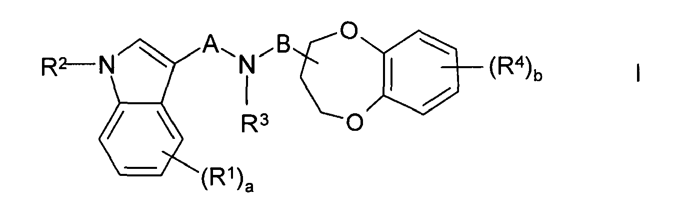 in which
R¹, independently of one another, is selected from alkyl, (CH₂)ₘOD, (CH₂)ₘCN, (CH₂)ₘCOR⁵ or (CH₂)ₘCH₂R⁵, where m=0 or 1,
R², R³, independently of one another, are selected from H, alkyl having 1 to 5 C atoms,
R⁴ independently of one another, is selected from alkyl having 1 to 5 C atoms, heteroalkyl having 1 to 5 C atoms, alkoxy having 1 to 5 C atoms, alkoxyalkyl having 2 to 5 C atoms, Hal, CN, COR⁵ or OH,
R⁵ stands for OD, NH₂, NHD or ND₂,
A stands for CₙH₂ₙ where n= 2, 3, or 4,
B stands for CₚH₂ₚ where p=0, 1, 2, 3 or 4,
D, independently of one another, is selected from H, alkyl having 1 to 5 C atoms, alkoxyalkyl having 2 to 5 C atoms, aryl or aralkyl
a, b stand for 0, 1 or 2 and
Hal stands for F, CI, Br or I
and physiologically tolerated salts and solvates thereof.

2. Benzodioxepines of the formula I according to Claim 1, **characterised in that** the radicals R² and R³ stand for H.

3. Benzodioxepines of the formula I according to Claim 1 or 2, **characterised in that** the radicals R² and R³ stand for H and at least one radical R¹ stands for (CH₂)ₘCN.

4. Benzodioxepines of the formula I according to Claim 1, 2 or 3, **characterised in that** a (CH₂)ₘCN is in the 5-position of the indole ring and a preferably stands for 1 and m preferably stands for 0.

5. Benzodioxepines of the formula I according to one of the preceding claims, **characterised in that** A stands for CₙH₂ₙ where n = 4 and B stands for CₚH₂ₚ where p = 1 or 0.

6. Benzodioxepines of the formula I according to one of the preceding claims, **characterised in that** R¹ stands for (CH₂)ₘCN in the 5-position of the indole ring and a stands for 1 and m stands for 0, R² and R³ stand for H, A stands for CₙH₂ₙ where n = 4 and B stands for CₚH₂ₚ where p = 1 or 0.

7. Benzodioxepines of the formula I according to one of the preceding claims selected from the group consisting of
N-(3,4-dihydro-2H-1,5-benzodioxepin-3-yl)-4-(5-cyano-3-indolyl)-butylamine,
3-{4-[7-methyl-3,4-dihydro-2H-1,5-benzodioxepin-3-ylamino]butyl}-indole-5-carbonitrile,
3-{4-(6-methyl-3,4-dihydro-2H-1,5-benzodioxepin-3-ylamino)butyl}-indole-5-carbonitrile,
3-[4-(6-methoxy-3,4-dihydro-2H-1,5-benzodioxepin-3-ylamino)butyl]-indole-5-carbonitrile and
3-[4-(3,4-dihydro-2H-1,5-benzodioxepin-3-yl)methylamino)butyl]-indole-5-carbonitrile.

8. Process for the preparation of benzodioxepines of the formula I according to one of Claims 1 to 7, **characterised in that**
a) a compound of the formula II 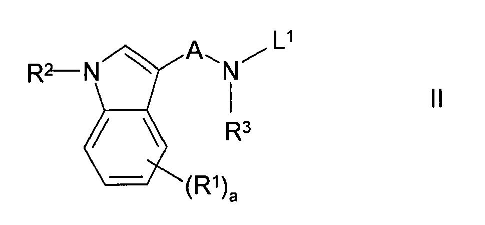 in which L¹ denotes H or a metal ion and R¹, R², R³, A and a have the meanings indicated in Claim 1,
b) is reacted with a compound of the formula III 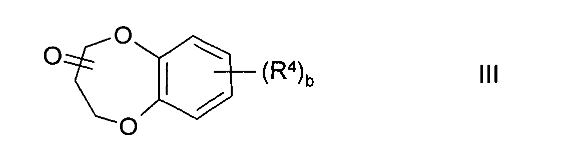 where R⁴ and b in formula III have the meanings indicated above and below in Claim 1, and optionally
c) a reduction step is optionally carried out and
d) the resultant compound of the formula I is optionally converted into one of its salts by treatment with an acid.

9. Process for the preparation of benzodioxepines of the formula I according to one of Claims 1 to 7 and physiologically tolerated salts and solvates thereof, **characterised in that**
a) a compound of the formula IV 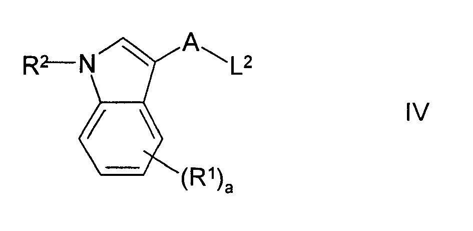 in which L² denotes CI, Br, I, OH, a reactively esterified OH group or a diazonium group and R¹, R², A and a have the meanings indicated in Claim 1,
b) is reacted with a compound of the formula V 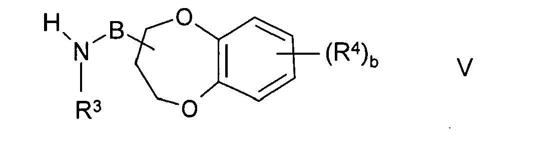 in which R³, R⁴, B and b have the meanings indicated in Claim 1, and optionally
c) the resultant compound of the formula I is converted into one of its salts by treatment with an acid.

10. Compounds of the formula I according to one of Claims 1 to 7 and/or physiologically tolerated salts and solvates thereof as medicaments.

11. Use of the compounds of the formula I according to one of Claims 1 to 7 for the preparation of a medicament for the treatment and/or prophylaxis of various diseases, such as, for example, strokes, cerebral ischaemia, and for reducing secondary damage caused by ischaemia, trauma, hypoglycaemia, schizophrenia, depression, dementia, dyskinesia, neurodegenerative diseases, such as Parkinson's disease, ALS, Alzheimer's disease, Lewy bodies dementia or Huntington's syndrome, Tourette's syndrome, anxiety, learning and memory impairment, sleeping disorders, inflammation-induced hyperalgesia, cerebral oedemas, under-supply states (hypoxia), inflammatory intestinal diseases and the associated disease symptoms, functional gastrointestinal diseases which are associated with pain and/or increased or reduced peristalsis, in particular irritable bowel syndrome, for the treatment and/or prophylaxis of non-ulcer-related dyspepsia, obstipation, in particular opioid-induced obstipation, arthritis, migraine, psoriasis or other irritative skin diseases, dysmenorrhoea, fibromyalgia, pain states, in particular pain oversensitivity reactions occurring in back complaints, burn injuries, sunburn and rheumatic diseases, postoperative pain and the ileus which frequently occurs after abdominal operations, diseases of the bladder, in particular irritable bladder, cytalgia, cystalgia, neuralgia or bladder neurosis.

12. Process for the preparation of a pharmaceutical composition, **characterised in that** at least one compound of the formula I according to one of Claims 1 to 7 and/or one of its physiologically tolerated salts or solvates is brought into a suitable dosage form together with at least one solid, liquid or semi-solid excipient or adjuvant.

13. Pharmaceutical composition, **characterised in that** it comprises an effective content of at least one compound of the formula I according to one of Claims 1 to 7 and/or one of its physiologically tolerated salts or solvates.

## Revendications

1. Benzodioxépines de la formule I 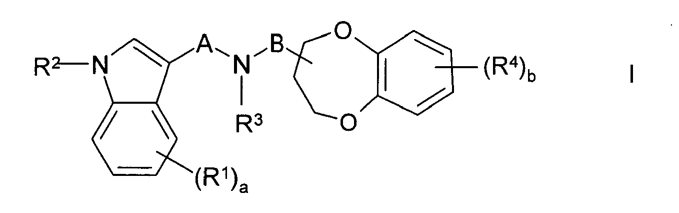 dans laquelle
R¹ indépendamment l'un de l'autre, est choisi parmi alkyle, (CH₂)mOD, (CH₂)ₘCN, (CH₂)ₘCOR⁵ ou (CH₂)ₘCH₂R⁵, où m=0 ou 1,
R², R³ indépendamment l'un de l'autre, sont choisis parmi H, alkyle comportant de 1 à 5 atomes de C,
R⁴ indépendamment l'un de l'autre, est choisi parmi alkyle comportant de 1 à 5 atomes de C, hétéroalkyle comportant de 1 à 5 atomes de C, alkoxy comportant de 1 à 5 atomes de C, alkoxyalkyle comportant de 2 à 5 atomes de C, Hal, CN, COR⁵ ou OH,
R⁵ représente OD, NH₂, NHD ou ND₂,
A représente CₙH₂ₙ où n= 2, 3 ou 4,
B représente CₚH₂ₚ où p=0, 1, 2, 3 ou 4,
D, indépendamment l'un de l'autre, est choisi parmi H, alkyle comportant de 1 à 5 atomes de C, alkoxyalkyle comportant de 2 à 5 atomes de C, aryle ou aralkyle
a, b représentent 0, 1 ou 2 et
Hal représente F, Cl, Br ou I
et leurs sels et solvates physiologiquement tolérés.

2. Benzodioxépines de la formule I selon la revendication 1, **caractérisés en ce que** les radicaux R² et R³ représentent H.

3. Benzodioxépines de la formule I selon la revendication 1 ou 2, **caractérisés en ce que** les radicaux R² et R³ représentent H et au moins un radical R¹ représente (CH₂)ₘCN.

4. Benzodioxépines de la formule I selon la revendication 1, 2 ou 3, **caractérisés en ce qu'**un (CH₂)ₘCN est à la position 5 de l'anneau indole et a représente de préférence 1 et m représente de préférence 0.

5. Benzodioxépines de la formule I selon l'une des revendications précédentes, **caractérisés en ce que** A représente CₙH₂ₙ où n = 4 et B représente CₚH₂ₚ où p = 1 ou 0.

6. Benzodioxépines de la formule I selon l'une des revendications précédentes, **caractérisés en ce que** R¹ représente (CH₂)ₘCN à la position 5 de l'anneau indole et a représente 1 et m représente 0, R² et R³ représentent H, A représente CₙH₂ₙ où n = 4 et B représente CₚH₂ₚ où p = 1 ou 0.

7. Benzodioxépines de la formule I selon l'une des revendications précédentes, choisis parmi le groupe comprenant
N-(3,4-dihydro-2H-1,5-benzodioxépine-3-yl)-4-(5-cyano-3-indolyl)-butylamine,
3-{4-[7-méthyl-3,4-dihydro-2H-1,5-benzodioxépine-3-ylamino]butyl}-indole-5-carbonitrile,
3-{4-(6-méthyl-3,4-dihydro-2H-1,5-benzodioxépine-3-ylamino)butyl}-indole-5-carbonitrile,
3-[4-(6-méthoxy-3,4-dihydro-2H-1,5-benzodioxépine-3-ylamino)butyl]-indole-5-carbonitrile et
3-[4-(3,4-dihydro-2H-1,5-benzodioxépine-3-yl)méthylamino)butyl]-indole-5-carbonitrile.

8. Procédé pour la préparation de benzodioxépines de la formule I selon l'une des revendications 1 à 7, **caractérisé en ce que**
a) un composé de la formule II 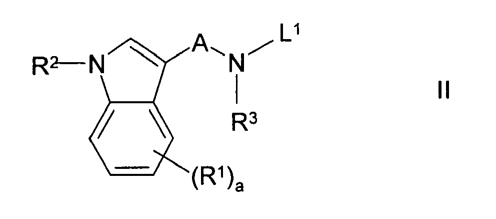 dans laquelle L¹ représente H ou un ion métal et R¹, R², R³, A et a présentent les significations indiquées dans la revendication 1,
b) est amené à réagir avec un composé de la formule III 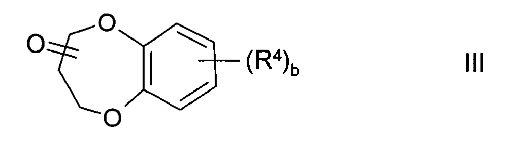 où R⁴ et b dans la formule III présentent les significations indiquées ci avant et ci après dans la revendication 1, et en option
c) une étape de réduction est en option mise en oeuvre et
d) le composé résultant de la formule I est en option converti selon l'un de ses sels par traitement avec un acide.

9. Procédé pour la préparation de benzodioxépines de la formule I selon l'une des revendications 1 à 7 et de leurs sels et solvates physiologiquement tolérés, **caractérisé en ce que**
a) un composé de la formule IV 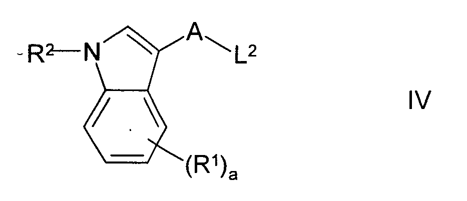 dans laquelle L² représente Cl, Br, I, OH, un groupe OH estérifié réactivement ou un groupe diazonium et R¹, R², A et a présentent les significations indiquées selon la revendication 1,
b) est amené à réagir avec un composé de la formule V 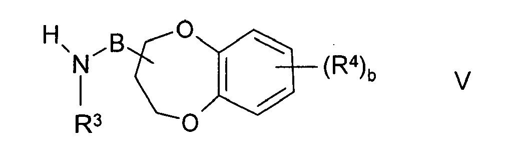 dans laquelle R³, R⁴, B et b présentent les significations indiquées dans la revendication 1, et en option
c) le composé résultant de la formule I est converti selon l'un de ses sels par traitement avec un acide.

10. Composés de la formule I selon l'une des revendications 1 à 7 et/ou leurs sels et solvates physiologiquement tolérés en tant que médicaments.

11. Utilisation des composés de la formule I selon l'une des revendications 1 à 7 pour la préparation d'un médicament pour le traitement et/ou la prophylaxie de diverses maladies telles que, par exemple, les accidents cérébrovasculaires, l'ischémie cérébrale, et pour réduire les dommages secondaires générés par l'ischémie, le trauma, l'hypoglycémie, la schizophrénie, la dépression, la démence, la dyskinésie, les maladies neurodégénératives telles que la maladie de Parkinson, la SLA, la maladie d'Alzheimer, la démence des corps de Lewy ou le syndrome de Huntington, le syndrome de Tourette, l'anxiété, l'altération de l'apprentissage et de la mémoire, les troubles du sommeil, l'hyperalgésie induite par inflammation, les oedèmes cérébraux, les états d'insuffisance (hypoxie), les maladies inflammatoires de l'intestin et les symptômes de maladie associés, les maladies gastro-intestinales fonctionnelles qui sont associées à la douleur et/ou le péristaltisme augmenté ou réduit, en particulier le syndrome d'irritation de l'intestin, pour le traitement et/ou la prophylaxie de la dyspepsie non liée aux ulcères, l'obstipation, en particulier l'obstipation induite par opioïde, l'arthrite, la migraine, du psoriasis ou d'autres maladies d'irritation de la peau, la dysménorrhée, la fibromyalgie, les états de douleur, en particulier les réactions de sursensibilité à la douleur rencontrées lors d'affections du dos, les blessures par brûlures, les maladies rhumatismales et causées par les brûlures du soleil, la douleur post-opératoire et l'occlusion intestinale rencontrée fréquemment après des opérations abdominales, les maladies de la vessie, en particulier l'irritation de la vessie, la cytalgie, la cystalgie, la neuralgie ou la neurose de la vessie.

12. Procédé pour la préparation d'une composition pharmaceutique, **caractérisé en ce qu'**au moins un composé de la formule I selon, l'une des revendications 1 à 7 et/ou l'un de ses sels ou solvates physiologiquement tolérés sont amenés selon une forme de dosage appropriée en association avec au moins un excipient ou adjuvant solide, liquide ou semi-solide.

13. Composition pharmaceutique, **caractérisée en ce qu'**elle comprend une teneur efficace en au moins un composé de la formule I selon l'une des revendications 1 à 7 et/ou en l'un de ses sels ou solvates physiologiquement tolérés.
